# EUROPEAN PATENT SPECIFICATION

(11) **EP 2 859 347 B1**
(45) Date of publication and mention of the grant of the patent: **24.05.2017**
(21) Application number: 13726804.1
(22) Date of filing: 05.06.2013
(51) Int. Cl.: G01N 33/573

(54) **DETECTION METHOD FOR THE IDENTIFICATION OF INSECTICIDE RESISTANCE**
DETEKTIONSVERFAHREN ZUR IDENTIFIZIERUNG VON INSEKTIZIDRESISTENZ
PROCÉDÉ DE DÉTECTION POUR L'IDENTIFICATION DE LA RÉSISTANCE À DES INSECTICIDES

(30) Priority: 08.06.2012 EP 12004357; 08.06.2012 US 201261657173 P; 12.09.2012 EP 12184005
(43) Date of publication of application: 15.04.2015
(73) Proprietor: Bayer CropScience AG, 40789 Monheim am Rhein (DE)
(72) Inventor: NAUEN, Ralf, 40764 Langenfeld (DE); RAMING, Klaus, 51375 Leverkusen (DE); WÖLFEL, Katharina, 40764 Langenfeld (DE)
(74) Representative: BIP Patents
(86) International application number: PCT/EP2013/061619
(87) International publication number: WO 2013/182613

(56) References cited:
- WO-A2-03/025223
- EMMANOUIL RODITAKIS ET AL: "Assessment of the Bemisia tabaci CYP6CM1vQ transcript and protein levels in laboratory and field-derived imidacloprid-resistant insects and cross-metabolism potential of the recombinant enzyme", INSECT SCIENCE, vol. 18, no. 1, 1 February 2011 (2011-02-01), pages 23-29, XP055054008, ISSN: 1672-9609, DOI: 10.1111/j.1744-7917.2010.01384.x
- KARUNKER I ET AL: "Over-expression of cytochrome P450 CYP6CM1 is associated with high resistance to imidacloprid in the B and Q biotypes of Bemisia tabaci (Hemiptera: Aleyrodidae)", INSECT BIOCHEMISTRY AND MOLECULAR BIOLOGY, ELSEVIER SCIENCE LTD, GB, vol. 38, no. 6, 1 June 2008 (2008-06-01), pages 634-644, XP022686318, ISSN: 0965-1748, DOI: 10.1016/J.IBMB.2008.03.008 [retrieved on 2008-03-29] cited in the application
- BERGE J-B ET AL: "Cytochrome P450 monooxygenases and insecticide resistance in insects", PHILOSOPHICAL TRANSACTIONS. ROYAL SOCIETY OF LONDON.BIOLOGICAL SCIENCES, ROYAL SOCIETY, LONDON, GB, vol. 353, no. 1376, 29 October 1998 (1998-10-29), pages 1701-1705, XP008020773, ISSN: 0962-8436, DOI: 10.1098/RSTB.1998.0321
- RALF NAUEN ET AL: "Pymetrozine is hydroxylated by CYP6CM1, a cytochrome P450 conferring neonicotinoid resistance in Bemisia tabaci", PEST MANAGEMENT SCIENCE, vol. 69, no. 4, 16 April 2013 (2013-04-16) , pages 457-461, XP55301963, BOGNOR REGIS; GB ISSN: 1526-498X, DOI: 10.1002/ps.3460
- RALF NAUEN ET AL: "Flupyradifurone: a brief profile of a new butenolide insecticide", PEST MANAGEMENT SCIENCE, 1 October 2014 (2014-10-01), pages n/a-n/a, XP055154661, ISSN: 1526-498X, DOI: 10.1002/ps.3932

## Description

### Field of the invention

This invention relates to a method for controlling insect pests of the species *Bemisia tabaci.*

### Background art

It is known that resistance to the neonicotinoid insecticide imidacloprid in cotton whitefly (*Bemisia tabaci*) is associated with high expression levels of the cytochrome P450 monooxygenase CYP6CM1 (Insect Biochemistry and Molecular Biology 38, (2008), 634-644), (Insect Science 18 (2011) 23-29).

### Problem

A problem of growing importance in agriculture is the development of resistance to certain widely used insecticides in a variety of economically relevant insect pests (Pest Manag Sci 2009; 65: 313-322). The application of insecticides to insect pests, that have developed a certain level of resistance to these insecticides, results in crop losses and adds further selective pressure towards additional resistance buildup in these pest populations.

One of the preferred insecticides to control whitefly pests are neonicotinoid insecticides. Neonicotinoid insecticides comprise the following commercialized active ingredients: imidacloprid, thiacloprid, clothianidin, thiamethoxam, acetamiprid, nitenpyram, and dinotefuran. They are also named IRAC Mode of Action class (MoA class) 4A by the Insecticide Resistance Action Committee (IRAC; www.irac-online.org).

Although, farmers would preferably treat whitefly pests with neonicotinoid chemistry, they are faced with a growing number of insect populations that have developed at least some sort of resistance against these active ingredients. Switching fully to other insecticides from different chemistries or different IRAC MoA classes would most likely result in the development of populations of pests being resistant to these insecticides as well. In addition, other chemistries might be inferior in their insecticidal activity. The standard determination of resistance of pests to specific insecticides currently requires a laboratory environment. Under field or greenhouse conditions, farmers would have to try out an insecticide and then observe if it can control the particular pest of interest. However, the result would be obtained too late, as resistant insects would have already destroyed at least part of the crop. In addition, weak resistance, that already require a resistance management, are not visible under these conditions.

It would therefore be of great practical importance to have an easy to use system, the farmer could directly work in the field, which further achieves a high sensitivity and reliability, and would provide him with a result in minutes, so he can implement proper resistance management strategies based on the rotational use of different mode of action classes not affected by resistance mechanisms.

### Description

### Principle

Surprisingly, levels of cytochrome P450 monooxygenases, in particular CYP6CM1 in combination with an easy to use antibody detection system allow a fast, reliable, and very sensitive detection of resistance of insects, in particular *Bemisia tabaci,* to neonicotinoid and pymetrozine chemistry.

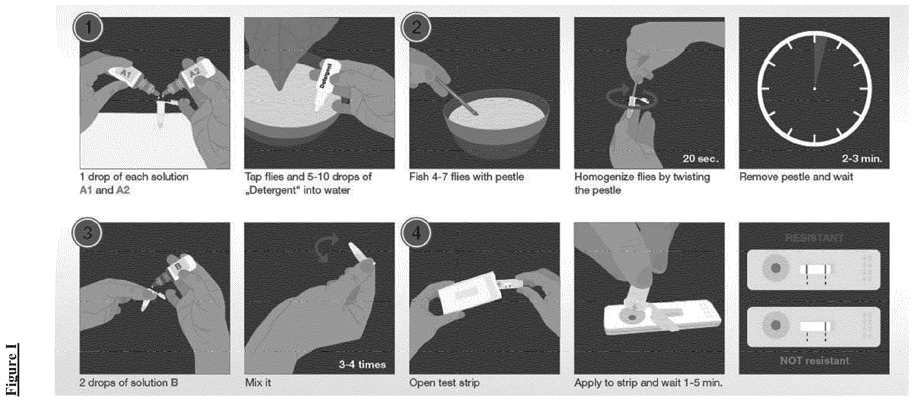

### The Test kit (see also Figure 1)

The basis of the test is the immunological detection, preferably via an ELISA assay, of a P450 monooxygenase, preferably CYP6CM1. The P450 monooxygenase protein is recognized by one or preferably by two different polyclonal antibodies (solutions A1 and A2). Particularly preferred is a combination of an antibody labeled with biotin and an additional antibody labeled with digoxygenin. In the presence of the P450 antigen, the biotinylated antibody will bind additionally to the streptavidin-gold solution (solution B) and form a complex, which now is detected by an anti-digoxygenin antibody spotted as a line on the test strip.

The kit is typically delivered with 3 solutions (A1, A2 and B), single use homogenization vials, single use homogenization pestles and sealed test strips.

The presence of neonicotinoid or pymetrozine resistance can typically be observed by treating 1-20 whiteflies in one whitefly test as described in the test procedure below. An optimum would be around four flies; more than 20 animals would result in a quenched signal, and thus be less preferred.

Whiteflies may be collected in a water bowl, e.g. a plant saucer (ideally of dark color) filled with water and 1-10 drops of detergent (either use commercial dishwashing detergent or 10% Tween20 supplied with the kit). Tapping of flies from the infested plant down into the bowl will collect whiteflies, which float on the surface of the liquid. Flies will stick to the surface of a homogenization pestle when dipped into the bowl.

A general test procedure involves the addition of 1-2 drops of each solution A1 and A2 (each with sufficient amount of the corresponding biotin or digoxygenated labeled antibody, in phosphate buffer and detergent) into the homogenization vial (Figure 1, Picture 1). Next, the whiteflies are "fished" as described above off the water surface of the bowl. The pestle with 1-20 whiteflies sticking to its tip is inserted into the homogenization vial with liquid (solutions A1 and A2 dropped in before) and twisted carefully in order to homogenize flies (Figure 1, Picture 2). Homogenization is completed when the solution turns yellowish and turbid. The solution can be used directly for the next step without changing the final result; however, an incubation time of 1-5 min will increase the final signal.

After homogenization, 2 drops of solution B (containing the liquid streptavidin-gold in phosphate buffer and detergent) are added to the vial and the resulting solution is gently mixed by inversion of the vial 3-4 times (Figure 1, Picture 3). Again, 1-5 min of waiting before proceeding to the next step will increase signal but not change overall result.

For resistance detection, the complete solution is poured from the vial into the sample cavity of the test strip (test strips delivered with the whitefly kit are sealed and have to be removed from the package first) (Figure 1, Picture 4). A red control line (at the position of "C" marked on the housing) will appear upon flowing of the liquid up the strip, latest after five minutes. Only if this line is present the strip was running properly.

If the control line is present, but no additional test line (at the position of "T" marked on the housing), the tested *Bemisia tabaci* population is not resistant to neonicotinoids or pymetrozine based on the over-expression of CYP6CM1.

If a clear line at "T" (Figure 1) is visible, the population is neonicotinoid (and pymetrozine) resistant and an alternative active ingredient of a different mode of action class has to be applied to control those populations.

The detection of the resistance marker can typically be performed with any antibody or antibody derived detection system. The antibody could be either a polyclonal antibody or a monoclonal antibody or a combination of more than one monoclonal antibody. Alternatively, any other molecular high affinity system as e.g. aptamers can be used to work the invention. Preferably, a polyclonal antibody is used. Preferably, combinations of more than one antibody are used for the detection.

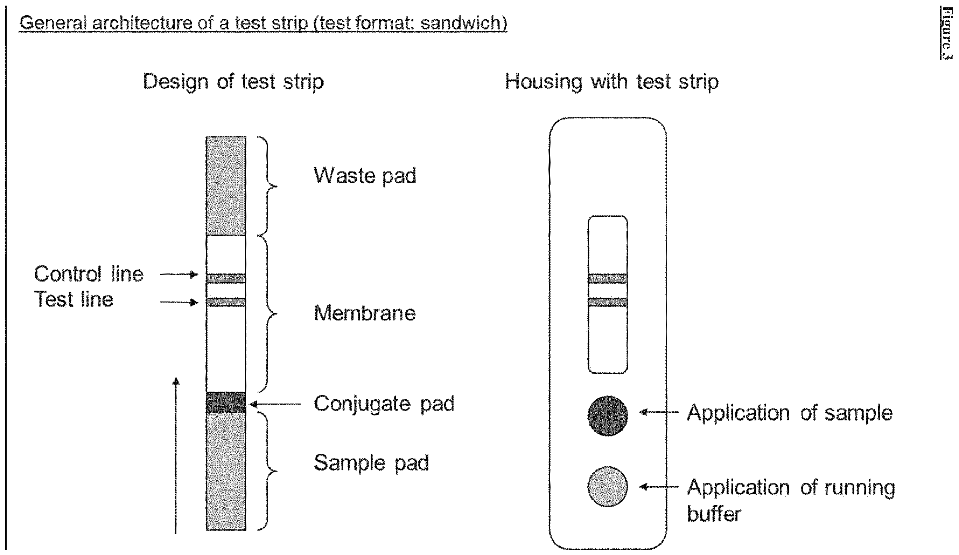

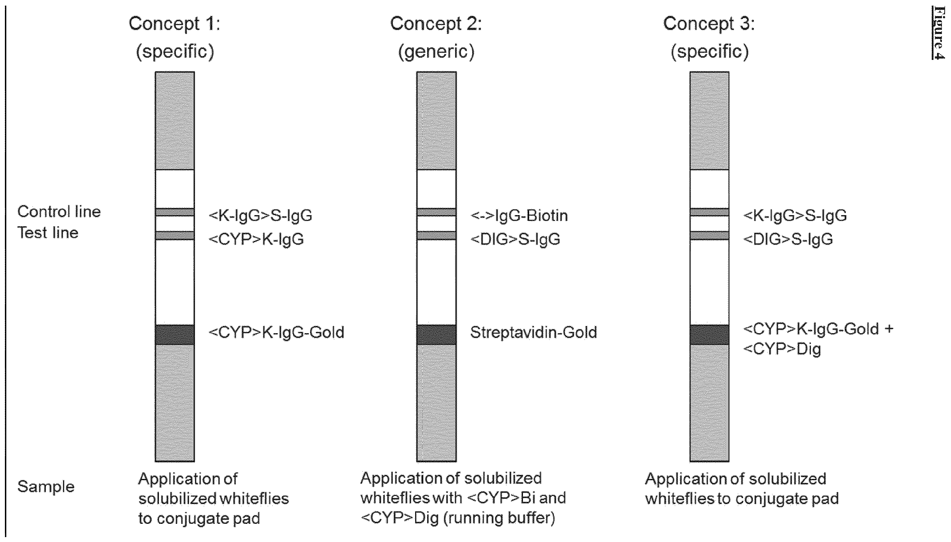

The detection of the resistance marker-antibody complex can typically be performed with any antibody detection system that allows a reasonably easy and quick signal generation as e.g. the digoxygenin detection system or the biotin-streptavidin system. Other suitable detection systems are based on the plasmon resonance principle. Preferably, a combination of a digoxygenin and a biotinylated antibody are preferred. The design of the detection system via a strip (lateral flow) based ELISA is exemplified in Figures 3 and 4.

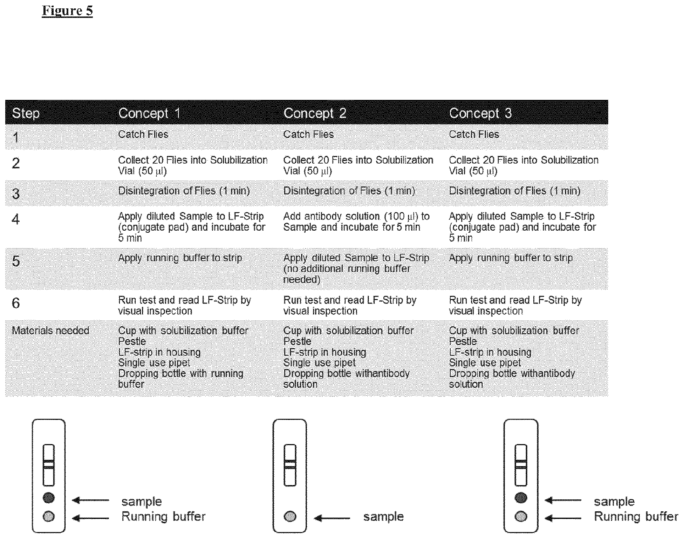

For illustration purposes, three different setups for the ELISA based detection of the resistance marker on strips are displayed in Figure 5. Preferably the detection of the antibody-resistance marker conjugates is performed via lateral flow analysis.

### The Method for Pest Control

The invention is a method as defined in independent claim 1. A further embodiment is disclosed in dependent claim 2. The resistance marker is CYP6CM1. The insect pest is the cotton whitefly (*Bemisia tabaci*).

The active ingredients specified herein by their "common name" are known and described, for example, in the Pesticide Manual ("The Pesticide Manual", 14th Ed., British Crop Protection Council 2006) or can be searched in the internet (e.g. http://www.alanwood.net/pesticides).

### Preparation and composition of reagents:

### Immunization of rabbits:

4 rabbits were immunized with His6-CYP6CM1 in PBS (500 µg per injection).

The immunization of a SPF rabbit (SPF=Specific Pathogen Free; strain: New Zealand White) includes:
- Immunization standard protocol (4 injections at day 0, 28, 42, 56), 2 control (after 40 and 54 days)
- Serum of pre-bleed (before first injection)
- Immunization is carried out by subcutaneous injections in combination with adjuvants (first injection CFA, second and further injections IFA)
- Delivery of serum (1. and 2. bleed 3-5 mL each, at day 70 bleed of about 20 ml each, then every month bleed of about 20 ml of each rabbit)

### Purification of antibodies:

The antibody was purified from serum by positive immunosorption. To prepare the column matrix, the antigen MBP-CYP6CM1 was coupled to NHS-activated Sepharose 4FF (*GE Healthcare*) following the instructions of the manufacturer. The purification was performed by binding the portion of antibody specific to CYP6CM1 by pumping the serum through a column filled with MBP-CYP6CM1-Sepharose. After two washing steps with (1) 0.5 M NaCl, 0.05% Tween 20 and (2) 30 mM NaCl the specific antibody was eluted under acidic conditions with a buffer containing 100 mM glycine, 0,5 M NaCl, pH 2.7. The antibody was dialyzed against PBS and concentrated by ultrafiltration.

### Modification of antibodies:

The antibodies were labelled by coupling NHS-activated labelling reagents that reacts efficiently with primary amino groups (-NH2) to form stable amide bonds. Antibodies have several primary amines in the side chain of lysine (K) residues and the N-terminus of each polypeptide that are available as targets for labeling with NHS-activated reagents. Out of these available amino groups, some residues are randomly coupled during reaction. The degree of labelling is dependent on the molar ratio used during conjugation reaction.

The biotinylation was performed in 100 mM phosphate buffer pH 8.5 with 2 to 8-fold molar excess of biotinylation reagent (succinimidyl-6-(biotinamido)hexanoate) at 25°C for two hours. The reaction was stopped by adding 10 mM lysine. The conjugate was purified from excess biotinylation reagent and by-products by dialysis against PBS. The degree of labelling was determined by a HABA assay.

The conjugation with Digoxigenin was performed in 100 mM phosphate buffer pH 8,5 with 8-fold molar excess of labeling reagent (Digoxigenin-3-O-methylcarbonyl-e-aminocaproic acid-N-hydroxysuccinimide ester) at 25°C for two hours. The reaction was stopped by adding 10 mM lysine. The conjugate was purified from excess labelling reagent and by-products by dialysis against PBS.

**Homogenisation Buffer:**

| **Ingredient** | **Range** | **Particularly preferred** |
|---|---|---|
| Sodium Phosphate or any other buffer system for a pH range of 6.0 to 8.0 | 0.1-10 mM, pH 6.0 to 8.0 | 1 mM, pH 6.7 |
| Detergent 1: e.g. Triton X-100 | 0,02 - 0.5 % per weight | 0.1 % per weight |
| Detergent 2: Dodecyl-β-D-maltosid (DDM), alternatively: n-Octyl- β-D-glucopyranosid (OG), 3-[(3-Cholamidopropyl)dimethylammonio]-1-propansulfonat (CHAPS), sodiumcholate, Polyethylenglykol 40000 (PEG40) | 0.5 - 3 % per weight | DDM 1% per weight |
| Optional: Saccharose or any other suitable reagent to control the osmolarity | 0 - 250 mM | 150 mM |

**Running Buffer:**

| **Ingredient** | **Range** | **Particularly preferred** |
|---|---|---|
| Sodium Phosphate or any other buffer system for a pH range of pH 7.5 to 9.0 | 0.1-10 mM, pH 7.5 to 9.0 | 1 mM, pH 8.0 |
| Detergent 1: e.g. Triton X-100 | 0,01 - 0.2 % per weight | 0.05 % per weight |
| Bovine Serum Albumine or any other appropriate agent to prevent unspecific binding | 0.05 - 1% per weight | 0.1% per weight |
| Optional: Saccharose or any other suitable reagent to control the osmolarity | 0 - 250 mM | 150 mM |

**Antibody:** 50 ng - 500 ng, preferably 150 ng each (Antibody biotin and antibody digoxygenin conjugate)
**Streptavidin-Gold** (40 nm): 100 mOD 50-300 mOD
**Teststrip for lateral flow application (see also** **Figures 3** **and** **4****):**
Membrane card: e.g. HF120 Plus (Millipore)
Waste Pad: C083 (Millipore) or any other similar cellulosepads.
Sample Pad: C083 (Millipore), treated with a solution comprising:
   a) 100 -400 mM Borate containing buffer, pH 8.0-10.0, preferably 250 mM sodium borate, pH 9.0;
   b) 0.1 - 3% per weight BSA, preferably 1% per weight BSA;
   c) 0.01-0,2% per weight Triton X-100, preferably 0.05% Triton X-100

### Example:

By utilization of the detection kit, a set of *Bemisia tabaci* strains was tested and susceptibility/resistance detected in the laboratory:
1) ESP-07M: resistant, Q-type, Spain
2) ESP-00: resistant, less resistant than 1), Q-type, Spain
3) SuS: susceptible, neither B nor Q-type, Sudan
4) B-Ref: susceptible, B-type, Israel
5) Crete: resistant, less resistant than 1) and 2), Q-type, Greece
6) EAE: resistant, B-type, Brazil

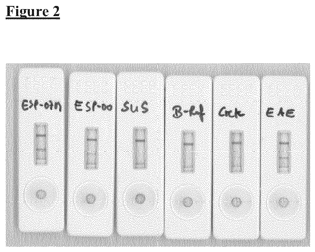

In this test strains 1, 2, 5 and 6 were positively tested for the resistance marker by using the kit (Figure 2). In all cases, the detection of the resistance marker correlated with the resistance against imidacloprid.

## Claims

1. Method for controlling insect pests comprising the following steps:
a. Obtaining a sample of the insect pest, wherein such insect pest is of the species *Bemisia tabaci*,
b. Performing an antibody based detection of a resistance marker which is CYP6CM1,
c. If the resistance marker is detected select an insecticide from the list A) consisting of spirotetramat, spiromesifen and flupyradifurone,
d. If the resistance marker is not present, then select an insecticide either from list A) or from the list B) comprised of: imidacloprid or pymetrozine.

2. Method according to claim 1, wherein list A) consists of spirotetramat and spiromesifen.

## Patentansprüche

1. Verfahren zum Bekämpfen von Schadinsekten, umfassend die folgenden Schritte:
a. Gewinnen einer Probe des Schadinsekts, wobei das Schadinsekt zu der Art *Bemisia tabaci* gehört,
b. Durchführen eines antikörperbasierten Nachweises eines Resistenzmarkers, bei dem es sich um CYP6CM1 handelt,
c. wenn der Resistenzmarker detektiert wird, Auswählen eines Insektizids aus der Liste A), die aus Spirotetramat, Spiromesifen und Flupyradifuron besteht,
d. wenn der Resistenzmarker nicht vorhanden ist, dann Auswählen eines Insektizids aus entweder Liste A) oder aus Liste B), die aus Imidacloprid oder Pymetrozin besteht.

2. Verfahren nach Anspruch 1, wobei die Liste A) aus Spirotetramat und Spiromesifen besteht.

## Revendications

1. Procédé de lutte contre les insectes nuisibles comprenant les étapes suivantes :
a. obtention d'un échantillon de l'insecte nuisible, un tel insecte nuisible étant de l'espèce *Bemisia tabaci*,
b. conduite d'une détection à base d'anticorps d'un marqueur de résistance qui est CYP6CM1,
c. si le marqueur de résistance est détecté, sélection d'un insecticide de la liste A) constituée des spirotétramat, spiromésifène et flupyradifurone,
d. si le marqueur de résistance n'est pas présent, alors sélection d'un insecticide de la liste A) ou de la liste B) constituée de : l'imidaclopride ou la pymétrozine.

2. Procédé selon la revendication 1, dans lequel la liste A) est constituée du spirotétramat et du spiromésifène.
